# EUROPÄISCHE PATENTSCHRIFT

(11) **EP 1 432 675 B1**
(45) Veröffentlichungstag und Bekanntmachung des Hinweises auf die Patenterteilung: **07.12.2005**
(21) Anmeldenummer: 02800124.6
(22) Anmeldetag: 26.09.2002
(51) Int. Cl.: C07C 217/58

(54) **VERFAHREN ZUR HERSTELLUNG VON VANILLYLAMIN HYDROCHLORID**
METHOD FOR PRODUCING VANILLYLAMINE HYDROCHLORIDE
PROCEDE DE PRODUCTION D'HYDROCHLORURE DE VANILLYLAMINE

(30) Priorität: 28.09.2001 DE 10147958
(43) Veröffentlichungstag der Anmeldung: 30.06.2004
(73) Patentinhaber: Boehringer Ingelheim Pharma GmbH & Co.KG, 55216 Ingelheim am Rhein (DE)
(72) Erfinder: MEYER, Oliver, 55452 Dorsheim (DE); HEDDESHEIMER, Ingo, 55569 Monzingen (DE)
(86) Internationale Anmeldenummer: PCT/EP2002/010803
(87) Internationale Veröffentlichungsnummer: WO 2003/029208

(56) Entgegenhaltungen:
- DE-C- 760 746
- ANU R. BAIPAI ET AL: "An efficient one-pot synthesis of armomatic nitriles from aldehydes using Fe modified K10" SYNTHETIC COMMUNICATION, Bd. 30, Nr. 15, 2000, Seiten 2785-2791, XP009003066

## Beschreibung

Die Erfindung betrifft ein großtechnisch anwendbares Verfahren zur Herstellung von Vanillylamin Hydrochlorid.

### Hintergrund der Erfindung

Vanillylamin Hydrochlorid ist ein Zwischenprodukt in der Herstellung von Pelargonsäurevanillylamid, welches als hyperämisierender Wirkstoff z.B. in Pflastern verwendet wird.
Das deutsche Patent DE 760 746 beschreibt die Herstellung von Vanillylamin Hydrochlorid, worin ausgehend von dem isolierten 4-Hydroxy-3-methoxybenzaldoxim dieses mittels Wasserstoff in Gegenwart von Aktivkohle und Palladiumoxid in essigsaurer Lösung und anschließender Salzsäurezugabe zum Vanillylamin Hydrochlorid reduziert wird.
In der Literatur (J. Org. Chem. Vol. 53, No. 5, 1988) wird die Herstellung des 4-Hydroxy-3-methoxy-benzaldoxim aus Vanillin und Hydroxylamin Hydrochlorid in basischem Medium beschrieben. Das Oxim wird zunächst isoliert und anschließend in salzsaurer ethanolischer Lösung zum Amin reduziert. Die Isolierung des Oxims bedeutet insbesondere im großtechnischen Verfahren einen entscheidenden Kosten- und Zeitaufwand in der Herstellung des Vanillylamin Hydrochlorids.
Es ist daher die Aufgabe der vorliegenden Erfindung ein großtechnisch anwendbares, kostengünstiges Verfahren zur Herstellung von Vanillylamin Hydrochlorid bereitzustellen.

### Detaillierte Beschreibung der Erfindung

Die vorliegende Erfindung löst die vorstehend genannte Aufgabe über das im folgenden beschriebene Syntheseverfahren.
Gegenstand der Erfindung ist somit ein Verfahren zur Herstellung von Vanillylamin oder eines seiner Salze durch
a) Umsetzung von Vanillin mit Hydroxylamin oder dessen Salzen in Gegenwart eines organischen Salzes, welches gegebenenfalls in situ erzeugt werden kann, und
b) anschließende Hydrierung des entstandenen Vanillyloxims mit Wasserstoff in Gegenwart eines geeigneten Katalysators und einer organischen und/oder anorganischen Säure, worin Schritt a) in einer anorganischen oder organischen Säure als Verdünnungsmittel durchgeführt wird.

Bevorzugt ist ein Verfahren, worin die Schritte a) und b) in einem Eintopfverfahren durchgeführt werden.

Weiterhin bevorzugt ist ein Verfahren, worin in Schritt a) Natriumacetat und Eisessig eingesetzt wird.

Besonders bevorzugt ist ein Verfahren, worin in Schritt a) Vanillin mit Hydroxylamin Hydrochlorid umgesetzt wird.

Weiterhin besonders bevorzugt ist ein Verfahren, worin in Schritt b) Pd/C oder Pt/C als Katalysator eingesetzt wird.

Erfindungsgemäß von besonderer Bedeutung ist ein Verfahren, in dem der Hydrierungsschritt b) in Gegenwart von Eisessig und konzentrierter Salzsäure erfolgt.

Die Erfindung betrifft vorzugsweise ein Verfahren, worin die Reaktionstemperatur in Schritt a) 15 °C bis 50 °C beträgt.

Die Erfindung betrifft bevorzugt ein Verfahren, worin die Reaktionstemperatur in Schritt b) 0°C bis 70 °C beträgt.

Insbesondere bevorzugt ist ein Verfahren, worin Vanillin zu Hydroxylamin oder dessen Salz in einem molaren Verhältnis von 1:2 bis 2:1, , vorzugsweise 1:1, eingesetzt wird.

Ein weiterer Gegenstand der Erfindung ist die Verwendung des nach dem erfindungsgemäßen Verfahren erhaltenen Vanillylamins oder eines seiner Salze zur Herstellung pharmazeutisch wirksamer Verbindungen.

Ein weiterer Gegenstand der Erfindung ist die Verwendung des nach dem erfindungsgemäßen Verfahren erhaltenen Vanillylamins oder eines seiner Salze zur Herstellung von Pelargonsäurevanillylamid.

Zur Salzbildung des Vanillylamins oder Vanillyloxims geeignete Säuren sind beispielsweise Salzsäure, Essigsäure, Bromwasserstoffsäure, Schwefelsäure, Phosphorsäure, Salpetersäure, Bemsteinsäure, Oxalsäure, Malonsäure, Fumarsäure, Maleinsäure, Weinsäure, Zitronensäure, Ascorbinsäure und Methansulfonsäure, insbesondere Salzsäure, Schwefelsäure oder Essigsäure.

In einer bevorzugten Ausführungsform des erfindungsgemäßen Verfahrens zur Herstellung des Vanillylamins wird in der Regel ein Äquivalent Vanillin in 3 bis 20 Anteilen (bezogen auf das Gewicht an eingesetztem Vanillin), vorzugsweise etwa 5 Anteilen, eines sauren Verdünnungsmittel, vorzugsweise Eisessig, Salzsäure, oder Schwefelsäure, besonders bevorzugt Eisessig, suspendiert und mit 1 bis 2 Äquivalenten, vorzugsweise einem Äquivalent eines organischen Salzes, beispielsweise Natriumhydroxid oder Natriumacetat, vorzugsweise Natriumacetat, insbesondere bevorzugt wasserfreies Natriumacetat, versetzt.
Die Suspension wird unter Rühren mit 1 bis 2 Äquivalenten, vorzugsweise einem Äquivalent, Hydroxylamin oder einem Salz des Hydroxylamins, vorzugsweise Hydroxylamin Hydrochlorid oder Hydroxylamin Sulfat bevorzugt Hydroxylamin Hydrochlorid, versetzt. Das Reaktionsgemisch wird auf 25 °C bis 50 °C, vorzugsweise auf 28 °C bis 40°C, bevorzugt etwa 30 °C bis 35 °C erwärmt und 0,5 bis 8 Stunden, vorzugsweise etwa 3 Stunden gerührt.
Das entstandene Vanillyloxim bleibt in der Suspension und wird durch Zugabe von 0,1 bis 10 Anteilen (bezogen auf das Gewicht an eingesetztem Vanillin),vorzugsweise etwa 0,7 Anteilen, Säure, vorzugsweise Salzsäure oder Schwefelsäure, bevorzugt Salzsäure, und 1 bis 20 Gewichts-% (bezogen auf eingesetztes Vanillin), vorzugsweise 10 Gewichts-%, eines Katalysators, vorzugsweise eines Übergangsmetallkatalysator, bevorzugt eines Pd/C-, eines Pt- oder eines Ra-Ni-Katalysators, insbesondere bevorzugt eines Pd/C-Katalysators, unter Einleitung von Wasserstoff in das Reaktionsgemisch, bei einem Wasserstoffdruck von 1 bis 7 bar, vorzugsweise jedoch von 3 bis 5 bar, besonders bevorzugt 4 bar, und einer Temperatur von 0 °C bis 70 °C, vorzugsweise 10 °C bis 35 °C, bevorzugt etwa 10°C, hydriert.
Anschließend wird das entstandene Vanillylamin bzw. dessen Salz durch Zugabe von Wasser und gegebenenfalls Erwärmung auf 40°C bis 70°C, vorzugsweise etwa 60 °C, vollständig in Lösung gebracht. Der Katalysator wird abfiltriert. Das Filtrat wird, gegebenenfalls nach Abdestillation des eingesetzten Verdünnungsmittels, vorzugsweise Essigsäure, zur Lösung der Salze und des Vanillylamins bzw. dessen Salz auf 50°C bis 70°C, vorzugsweise etwa 60 °C, erwärmt und mit Wasser versetzt. Zur Fällung des Vanillylamin-Salzes wird das Reaktionsgemisch mit einer anorganischen oder organischen Säure, vorzugsweise Salzsäure, versetzt. Die entstandene Suspension wird abgekühlt und das Salz des Vanillylamins filtriert, , gegebenenfalls mit einem Lösungsmittel, vorzugsweise mit Aceton, gewaschen und getrocknet.

Die erfindungsgemäße Vorgehensweise führt zu einem kostengünstigen Verfahren mit einer hohen Raum-Zeit-Ausbeute bezüglich des Vanillylamins bzw. dessen Salze.

Die nachfolgenden Beispiele dienen der Illustration exemplarisch durchgeführter Verfahren zur Herstellung des Vanillylamins bzw. dessen Salze. Sie sind lediglich als mögliche, exemplarisch dargestellte Vorgehensweisen zu verstehen, ohne die Erfindung auf deren Inhalt zu beschränken.

### Beispiel 1

### a) Synthese von Vanillyloxim

56,58 g Vanillin werden in 283 ml Eisessig suspendiert und mit 32,02 g wasserfreiem Natriumacetat versetzt. Anschließend werden 28,29 g Hydroxylamin Hydrochlorid zugegeben, das Reaktionsgemisch unter Rühren auf 30 °C bis 35 °C erwärmt und 30 Stunden gerührt.

### b) Synthese von Vanillylamin

Das unter a) erhaltenen Reaktionsgemisch wird mit 38 ml Salzsäure und 6,2 g Pd/C versetzt. Unter Rühren wird bei 10 °C über 4 Stunden Wasserstoff bei einem Druck von 4 bar in das Reaktionsgemisch geleitet. Nach Zugabe von 71 ml Wasser wird auf 60 °C erwärmt und 1 Stunde gerührt. Der Katalysator wird abfiltriert und die Essigsäure bei 60 °C destillativ aus dem Filtrat entfernt. Anschließend wird das Reaktionsgemisch zur Lösung des Vanillylamin Hydrochlorids und der Salze mit 141 ml Wasser versetzt und bei 60°C 0,5 Stunden gerührt. Nach Zugabe von 99 ml Salzsäure und 1 Stunde Rühren wird die entstandene Suspension auf 3 °C abgekühlt und nach 3 Stunden das Vanillylamin Hydrochlorid abfiltriert, mit Aceton gewaschen und bei 50 °C getrocknet.
Ausbeute: 57,66 g (82 % d. Th.)

### Beispiel 2

### a) Synthese von Vanillyloxim

20,0 g Vanillin werden in 150 ml Eisessig suspendiert und mit 8,6 ml NaOH (50%) versetzt. Anschließend werden 10,96 g Hydroxylamin Hydrochlorid zugegeben. Das Reaktionsgemisch wird 2 Stunden bei 20 °C gerührt.

### b) Synthese von Vanillylamin Hydrochlorid

Das unter a) erhaltene Reaktionsgemisch wird mit 27 ml Salzsäure und 2 g Pd/C versetzt. Unter Rühren wird bei 0 °C über 2,5 Stunden Wasserstoff bei einem Druck von 4 bar in das Reaktionsgemisch geleitet. Nach Aufarbeitung werden 13,12 g (53 % d. Th.) Vanillylamin Hydrochlorid erhalten.

## Patentansprüche

1. Verfahren zur Herstellung von Vanillylamin oder eines seiner Salze durch
a) Umsetzung von Vanillin mit Hydroxylamin oder dessen Salzen in Gegenwart eines organischen Salzes, welches gegebenenfalls in situ erzeugt werden kann, und
b) anschließende Hydrierung des entstandenen Vanillyloxims mit Wasserstoff in Gegenwart eines geeigneten Katalysators und einer organischen und/oder anorganischen Säure, **dadurch gekennzeichnet, dass** Schritt a) in einer anorganischen oder organischen Säure als Verdünnungsmittel durchgeführt wird.

2. Verfahren nach Anspruch 1, **dadurch gekennzeichnet, dass** die Schritte a) und b) in einem Eintopfverfahren durchgeführt werden.

3. Verfahren nach Anspruch 1 oder 2, **dadurch gekennzeichnet, dass** in Schritt a) Natriumacetat und Eisessig eingesetzt wird.

4. Verfahren nach einem der Ansprüche 1 bis 3, **dadurch gekennzeichnet, dass** in Schritt a) Vanillin mit Hydroxylamin Hydrochlorid umgesetzt wird.

5. Verfahren nach einem der Ansprüche 1 bis 4, **dadurch gekennzeichnet, dass** in Schritt b) Pd/C oder Pt/C als Katalysator eingesetzt wird.

6. Verfahren nach einem der Ansprüche 1 bis 5, **dadurch gekennzeichnet, dass** der Hydrierungsschritt b) in Gegenwart von Eisessig und konzentrierter Salzsäure erfolgt.

7. Verfahren nach einem der Ansprüche 1 bis 6, **dadurch gekennzeichnet, dass** die Reaktionstemperatur in Schritt a) 15 °C bis 50 °C beträgt.

8. Verfahren nach einem der Ansprüche 1 bis 6, **dadurch gekennzeichnet, dass** die. Reaktionstemperatur in Schritt b) 0°C bis 70° C beträgt.

9. Verfahren nach einem der Ansprüche 1 bis 8, **dadurch gekennzeichnet, dass** Vanillin zu Hydroxylamin oder dessen Salz in einem molaren Verhältnis von 1:2 bis 2:1 eingesetzt wird.

10. Verwendung des nach einem Verfahren gemäß einem der Ansprüche 1 bis 9 erhaltenen Vanillylamins oder eines seiner Salze zur Herstellung pharmazeutisch wirksamer Verbindungen.

11. Verwendung des nach einem Verfahren gemäß einem der Ansprüche 1 bis 9 erhaltenen Vanillylamins oder eines seiner Salze zur Herstellung von Pelargonsäurevanillylamid.

## Claims

1. Process for preparing vanillylamine or one of the salts thereof by
a) reacting vanillin with hydroxylamine or the salts thereof in the presence of an organic salt, which may optionally be produced *in situ,* and
b) subsequently hydrogenating the resulting vanillyloxime with hydrogen in the presence of a suitable catalyst and an organic and/or inorganic acid,
**characterised in that** step a) is carried out in an inorganic or organic acid as diluent.

2. Process according to claim 1, **characterised in that** steps a) and b) are carried out in a one-pot process.

3. Process according to claim 1 or 2, **characterised in that** in step a) sodium acetate and glacial acetic acid are used.

4. Process according to one of claims 1 to 3, **characterised in that** in step a) vanillin is reacted with hydroxylamine hydrochloride.

5. Process according to one of claims 1 to 4, **characterised in that** in step b) Pd/C or Pt/C is used as catalyst.

6. Process according to one of claims 1 to 5, **characterised in that** the hydrogenation step b) is carried out in the presence of glacial acetic acid and concentrated hydrochloric acid.

7. Process according to one of claims 1 to 6, **characterised in that** the reaction temperature in step a) is 15 °C to 50 °C.

8. Process according to one of claims 1 to 6, **characterised in that** the reaction temperature in step b) is 0°C to 70°C.

9. Process according to one of claims 1 to 8, **characterised in that** vanillin is used in a molar ratio to hydroxylamine or a salt thereof of 1:2 to 2:1.

10. Use of the vanillylamine or one of the salts thereof obtained by a process according to one of claims 1 to 9 for preparing pharmaceutically effective compounds.

11. Use of the vanillylamine or one of the salts thereof obtained by a process according to one of claims 1 to 9 for preparing pelargonic acid vanillylamide.

## Revendications

1. Procédé de production de vanillylamine ou de l'un de ses sels par
a) réaction de la vanilline avec l'hydroxylamine ou ses sels en présence d'un sel organique, qui peut éventuellement être produit in situ, et
b) hydrogénation subséquente de la vanillyloxime formée avec l'hydrogène en présence d'un catalyseur approprié et d'un acide organique et/ou inorganique, **caractérisé en ce que** l'étape a) est réalisée dans un acide inorganique ou organique comme diluant.

2. Procédé selon la revendication 1 **caractérisé en ce que** les étapes a) et b) sont réalisées dans un procédé à récipient unique.

3. Procédé selon la revendication 1 ou 2 **caractérisé en ce que** l'acétate de sodium et l'acide acétique glacial sont utilisés dans l'étape a).

4. Procédé selon l'une des revendications 1 à 3 **caractérisé en ce que** la vanilline est mise à réagir avec le chlorhydrate d'hydroxylamine dans l'étape a).

5. Procédé selon l'une des revendications 1 à 4 **caractérisé en ce que** Pd/C ou Pt/C est utilisé comme catalyseur dans l'étape b).

6. Procédé selon l'une des revendications 1 à 5 **caractérisé en ce que** l'étape d'hydrogénation b) a lieu en présence d'acide acétique glacial et d'acide chlorhydrique concentré.

7. Procédé selon l'une des revendications 1 à 6 **caractérisé en ce que** la température de réaction dans l'étape a) est 15°C à 50°C.

8. Procédé selon l'une des revendications 1 à 6 **caractérisé en ce que** la température de réaction dans l'étape b) est 0°C à 70°C.

9. Procédé selon l'une des revendications 1 à 8 **caractérisé en ce que** la vanilline est utilisée par rapport à l'hydroxylamine ou son sel dans un rapport molaire de 1 : 2 à 2 : 1.

10. Utilisation de la vanillylamine obtenue selon un procédé selon l'une des revendications 1 à 9 ou de l'un de ses sels pour la production de composés pharmaceutiquement efficaces.

11. Utilisation de la vanillylamine obtenue selon un procédé selon l'une des revendications 1 à 9 ou de l'un de ses sels pour la production de vanillylamide d'acide pélargonique.
